# FASCICULE DE BREVET EUROPEEN

(11) **EP 2 715 437 B1**
(45) Date de publication et mention de la délivrance du brevet: **10.01.2018**
(21) Numéro de dépôt: 12723701.4
(22) Date de dépôt: 25.05.2012
(51) Int. Cl.: A42B 3/22, A61F 9/02, G02F 1/133, G02C 7/10, A61F 9/06

(54) **FILTRE OPTIQUE ADAPTATIF**
ADAPTIVES OPTISCHES FILTER
ADAPTIVE OPTICAL FILTER

(30) Priorité: 25.05.2011 FR 1154562
(43) Date de publication de la demande: 09.04.2014
(73) Titulaire: Casbi, Evelyne, 75008 Paris (FR); Borreau, Jean-Paul, 75008 Paris (FR)
(72) Inventeur: Casbi, Evelyne, 75008 Paris (FR); Borreau, Jean-Paul, 75008 Paris (FR)
(74) Mandataire: Regimbeau
(86) Numéro de dépôt international: PCT/EP2012/059871
(87) Numéro de publication internationale: WO 2012/160196

(56) Documents cités:
- EP-A1- 0 917 865
- EP-A2- 0 341 519
- WO-A1-92/10130
- WO-A1-93/24858
- WO-A1-2008/148240
- WO-A1-2009/069166
- FR-A1- 2 373 808

## Description

### DOMAINE DE L'INVENTION

L'invention concerne le domaine des filtres optiques utilisés pour diminuer une intensité lumineuse, et plus particulièrement le domaine des filtres optiques comprenant des obturateurs optiques à cristaux liquides pouvant s'adapter à la luminosité ambiante.

L'invention peut être mise en oeuvre notamment dans des dispositifs de protection contre la lumière utilisés au quotidien comme des lunettes de soleil.

### ETAT DE LA TECHNIQUE

On connaît déjà de nombreux dispositifs permettant de diminuer une intensité lumineuse, dont certains comprennent des obturateurs à cristaux liquides et peuvent s'adapter à la luminosité ambiante.

On note en particulier le document FR 2,693,562, qui décrit des lunettes solaires comprenant un capteur photosensible, émettant un signal continu, fonction croissante de l'intensité lumineuse qui l'atteint, et un circuit électronique relié à des écrans à cristaux-liquides, tels que la transmittance des écrans diminue, c'est-à-dire que l'opacité augmente quand l'intensité lumineuse reçue par le capteur photosensible augmente.

On connaît également le document FR 2,781,289, qui reprend l'ensemble des caractéristiques du document FR 2, 693, 562 et dans lequel l'obscurcissement des verres est en outre proportionnel à l'intensité lumineuse reçue par le capteur photosensible.

On connaît encore le document PCT/US2007/019631, qui propose une solution dans laquelle une batterie alimente un circuit électronique de commande d'un obturateur à cristaux liquides. Ce circuit électronique est très complexe et présente donc un temps de réponse assez long.

Deux autres documents pertinentes de l'état de la technique sont les documents EP0341519 et US5654786.

Ces dispositifs présentent toutefois de nombreux inconvénients.

En premier lieu, l'obscurcissement des verres de ces dispositifs nécessite un temps d'adaptation de quelques dixièmes de seconde, voire de quelques secondes, ce qui les rend inadaptés à certaines situations, au cours desquelles l'obscurcissement doit avoir lieu de manière instantanée.

Parmi ces situations, on compte notamment des situations de déplacement par temps ensoleillé, en voiture ou en moto par exemple, au cours desquelles le conducteur passe par des tunnels, ou sous un couloir d'arbres, etc. En circonstances normales, le conducteur peut porter des lunettes de soleil ou un casque équipé d'une visière teintée, afin de ne pas être ébloui. Lorsqu'il atteint l'un de ces passages où des transitions rapides de la luminosité ont lieu, le conducteur peut enlever ses lunettes ou relever sa visière, mais cela le met en danger car cette transition lui coûte quelques secondes d'inattention et de non possession de tous ses moyens.

De plus, le conducteur prend aussi des risques s'il n'enlève pas ses lunettes ou sa visière, car il a alors une visibilité réduite.

Aucun des dispositifs adaptables évoqués ci-avant ne permet de résoudre ces problèmes puisque, au cours du temps de transition susmentionné, l'usager conserve une visibilité perturbée ou réduite, ce qui représente un risque pour lui comme pour les autres usagers.

En outre, pour les dispositifs utilisant des cristaux liquides, par exemple de type nématique, dont l'alimentation et continue, proposant un obscurcissement proportionné à la luminosité incidente, il arrive parfois que les molécules des cristaux liquides ne s'orientent pas de façon homogène selon l'orientation adaptée à l'obturation requise. Il en résulte des phénomènes optiques tels que des diaprures ou irisations sur les verres des lunettes, qui peuvent être gênants pour l'usager.

Une solution alternative, présentée dans le document WO2009/069166, propose un filtre optique dans lequel un capteur photosensible délivre une tension à un écran à cristaux liquides pour commander l'obturation de celui-ci. Le circuit électronique comprend également une résistance permettant de décharger rapidement les charges résiduelles dans l'écran à cristaux liquides afin que celui-ci ne présente pas les irisations mentionnées ci-avant.

Egalement, des solutions dans lesquelles une batterie supplémentaire délivre une tension hachée à l'obturateur ont été proposées, mais il existe toujours un risque que la batterie fasse défaut à l'utilisateur.

En conséquence, un but de la présente invention est de fournir une alternative à la solution précédente, et en particulier un filtre optique sécurisé pour l'usager, permettant une adaptation quasi-instantanée à l'intensité lumineuse incidente sur le filtre, et évitant tout phénomène optique gênant pour l'usager.

Un autre but de l'invention est de pouvoir être mise en oeuvre facilement sur un support tel que des lunettes, un casque, ou encore une fenêtre.

A cet effet, l'invention propose un filtre optique tel que défini en revendication 1 annexée.

Avantageusement, mais facultativement, le filtre optique proposé par l'invention comprend au moins l'une des caractéristiques suivantes :
- le filtre optique comprend en outre un régulateur de tension adapté pour limiter la tension appliquée à l'obturateur, positionné en aval de l'interrupteur, et adapté pour délivrer une tension Uₑ constante et strictement supérieure à la tension de polarisation U_{LCD} de l'obturateur, ou bien l'opacité OP₂ est constante pour toute tension Uₑ strictement supérieure à la tension de polarisation U_{LCD}
- le filtre optique comprend en outre un dispositif de réglage manuel de l'opacité OP₂ de l'obturateur à cristaux liquides,
- le filtre optique comprend en outre un dispositif de désactivation manuelle permanente du module électronique de commande,
- le capteur photosensible est intégré à au moins une surface d'appui, et le module électronique de commande est réalisé sur un circuit imprimé par sérigraphie sur au moins un verre,
- l'obturateur à cristaux liquides est intégré dans une zone partielle d'un verre.

L'invention concerne également une paire de lunettes et un casque équipés du filtre optique selon l'invention.

### DESCRIPTION DES FIGURES

D'autres caractéristiques, buts et avantages de la présente invention apparaîtront à la lecture de la description détaillée qui va suivre, au regard des figures annexées, données à titre d'exemples non limitatifs et sur lesquelles :
- les figures 1a et 1b représentent deux exemples de structures électroniques d'un filtre optique selon des modes de réalisation de l'invention,
- les figures 2a à 2c représentent des exemples de diagrammes de fonctionnement d'éléments du filtre optique en relation avec l'intensité lumineuse incidente sur le filtre,
- les figures 3a à 3c représentent schématiquement des exemples de structures physiques d'un filtre optique selon l'invention
- les figures 4a et 4b représentent des exemples d'intégration d'un filtre optique selon l'invention,
- la figure 5 représente un exemple d'intégration d'un filtre optique selon l'invention dans un verre de lunette à correction optique.

### DESCRIPTION DETAILLEE DE L'INVENTION

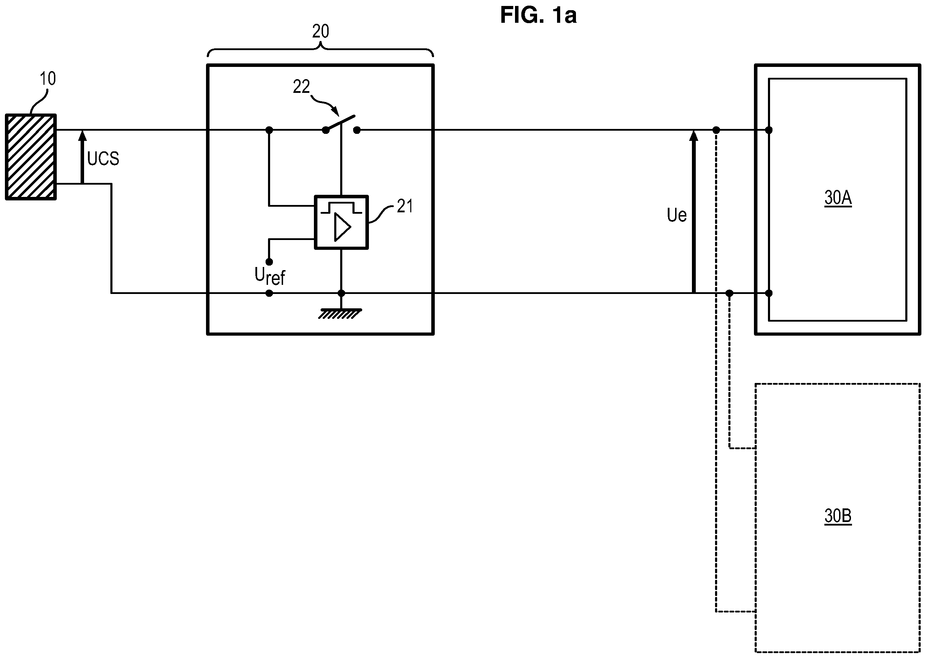

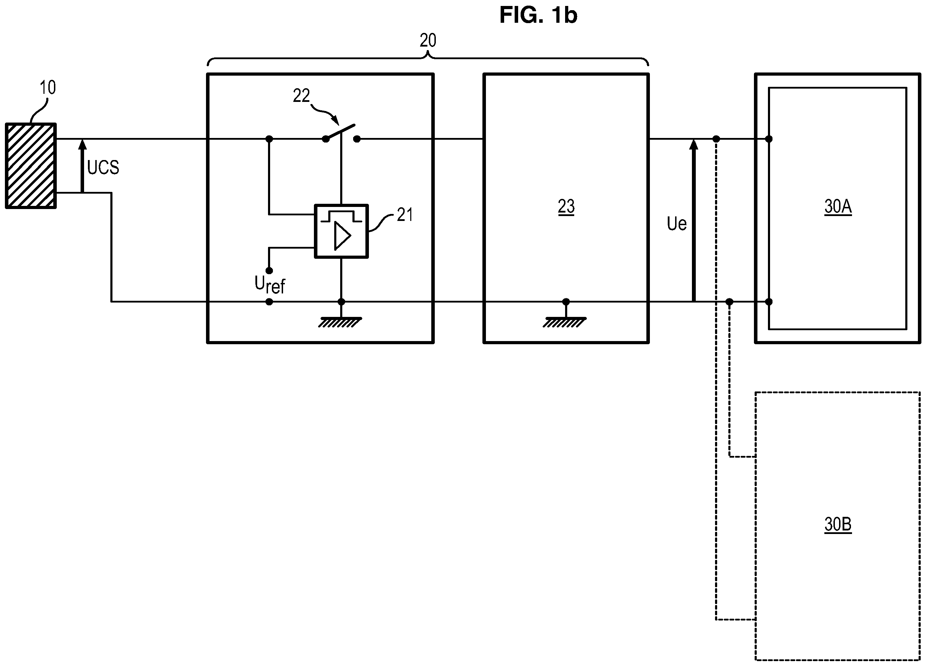

En référence aux figures 1a et 1b, sont représentés des exemples de structures électroniques selon des modes de réalisation préférés du filtre optique selon la présente invention.

Ce filtre optique 1 comprend de préférence :
- un capteur photosensible 10, par exemple une cellule photovoltaïque, adapté pour délivrer une tension U_{CS} variable en fonction de l'intensité lumineuse qu'il reçoit. La tension U_{CS} peut par exemple être continue et croissante en fonction de l'intensité lumineuse reçue par le capteur 10,
- un module électronique de commande 20,
- au moins un obturateur optique 30, de préférence à cristaux liquides.

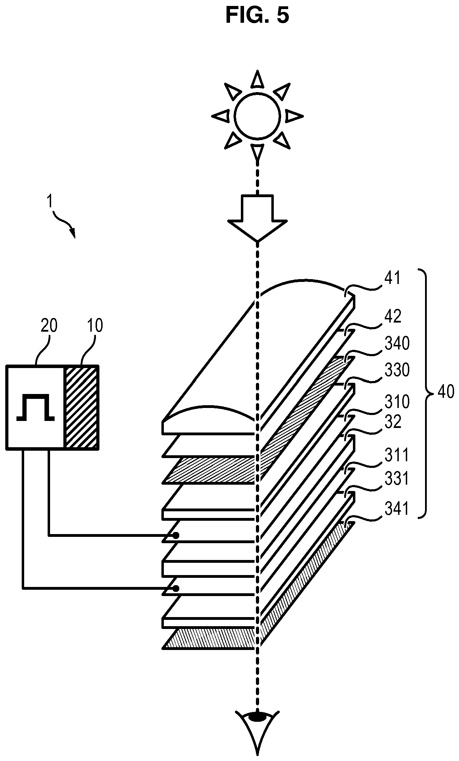

L'obturateur optique 30 est de type connu de l'état de la technique. Il s'agit par exemple d'un obturateur à cristaux liquides de type nématique. Le cas échéant, il comprend de préférence des électrodes 310, 311, une pellicule de cristaux liquides 32, des écrans optiquement transparents 330, 331 et des polariseurs 340, 341 (illustrés en figure 5).

De préférence, la pellicule de cristaux liquides 32 est positionnée entre deux électrodes 310 et 311, celles-ci étant elles-mêmes positionnées entre deux écrans optiquement transparents 330 et 331.

Enfin, l'obturateur optique 30 comprend avantageusement deux polariseurs 340 et 341, les deux polariseurs étant de préférence positionnés contre les surfaces externes des écrans optiquement transparents 330 et 331, de sorte que ceux-ci se trouvent entre les polariseurs. Alternativement, les polariseurs 340 et 341 peuvent se trouver entre une électrode 310 (resp. 311) et un écran 330 (resp. 331) correspondant, ou encore entre une électrode correspondante et la pellicule de cristaux liquides.

De manière connue de l'homme de l'art, les deux polariseurs 340, 341 sont typiquement orientés de 90° et les molécules nématiques présentent une orientation hélicoïdale quand aucune tension n'est appliquée, permettant à la lumière de passer sans être obturée. L'application d'une tension aux bornes de ces électrodes 310, 311 peut déclencher, selon la valeur de la tension, une orientation particulière des molécules nématiques, entraînant une opacification partielle ou totale de l'obturateur 30. Ainsi, si l'obturateur 30 est placé sur le trajet d'un faisceau lumineux incident, la quantité de lumière transmise varie en fonction de son opacité. En effet, l'opacité est ici définie comme l'inverse de la transmittance, celle-ci étant le ratio entre l'intensité lumineuse transmise au travers de l'obturateur et l'intensité lumineuse incidente. L'opacité est donc le ratio entre l'intensité lumineuse incidente et l'intensité lumineuse transmise.

L'obturateur optique 30 présente de préférence une tension de polarisation U_{LCD} formant un seuil entre deux états distincts, pour lesquels l'opacité de l'obturateur optique présente deux niveau différents.

Selon un mode de réalisation préféré de l'obturateur optique, la tension de polarisation U_{LCD} est telle que, lorsqu'il reçoit à ses bornes une tension inférieure à cette tension de polarisation, voire une tension nulle ou une absence de tension, les molécules nématiques sont orientées de manière à donner à l'obturateur une opacité particulière, par exemple inférieure ou égale à une première opacité OP₁. De préférence, pour une tension d'entrée aux bornes de l'obturateur 30 inférieure à U_{LCD}, l'opacité de l'obturateur est constante et égale à l'opacité OP₁. L'opacité OP₁ est par exemple très faible, c'est-à-dire de l'ordre de grandeur de l'opacité d'un verre transparent utilisé traditionnellement dans le domaine de la lunetterie.

A l'inverse, lorsque l'obturateur 30 reçoit à ses bornes une tension supérieure à cette tension de polarisation U_{LCD}, les molécules nématiques s'orientent de manière à ce que l'obturateur présente au moins une opacité OP₂ strictement supérieure à l'opacité OP₁.

Selon un mode de réalisation particulier de l'obturateur, celui-ci peut être un obturateur de type « tout ou rien », présentant une opacité OP₁ pour une tension d'entrée inférieure à la tension de polarisation U_{LCD}, ou nulle ou pour aucune tension appliquée, et une opacité OP₂ constante strictement supérieure à l'opacité OP₁ pour une tension d'entrée supérieure à la tension de polarisation U_{LCD}.

Selon un mode de réalisation alternatif, l'obturateur à cristaux liquides 30 peut présenter une opacité OP₂ variable, par exemple croissante en fonction de la tension d'entrée aux bornes de l'obturateur 30.

Selon l'invention, le module électronique de commande 20 délivre aux bornes 31 des électrodes 310, 311 de l'obturateur 30 une tension Uₑ.

Le module électronique de commande est lui-même électriquement relié au capteur photosensible 10, qui constitue sa seule source d'alimentation. Ainsi le capteur photosensible 10 délivre en sortie une tension U_{CS}, cette tension étant la tension d'entrée aux bornes du module électronique de commande 20.

Le module électronique de commande 20 comprend de préférence au moins un comparateur 21 et un interrupteur 22. Le comparateur 21 reçoit directement la tension U_{CS} délivrée par le capteur photosensible 10, et commande l'ouverture et la fermeture de l'interrupteur 22, cet interrupteur étant branché en série des bornes des électrodes 310, 311 de l'obturateur optique 30.

Ainsi, la seule source d'alimentation de l'obturateur optique 30 est le capteur photosensible 10. En particulier, quand le comparateur 21 commande l'ouverture de l'interrupteur 22, l'obturateur n'est pas alimenté, son opacité est donc inférieure ou égale à OP₁, et de préférence égale à OP₁.

Si le filtre optique comprend une pluralité d'obturateurs à cristaux liquides, ils sont alors branchés en parallèle sur la sortie du module électronique de commande 20 afin de recevoir aux bornes de leurs électrodes la même tension d'entrée.

Le principe de fonctionnement du filtre optique consiste en un fonctionnement à seuil sur l'intensité lumineuse incidente à l'obturateur à cristaux liquides 30. Si l'intensité lumineuse dépasse un certain seuil, par exemple un seuil d'éblouissement Iₑ, alors le module électronique de commande 20 délivre à l'obturateur 30 une tension strictement supérieure à la tension de polarisation U_{LCD}, afin que l'opacité de l'obturateur soit égale à au moins une opacité OP₂ strictement supérieure à l'opacité OP₁.

Un mode de réalisation préféré de l'invention est décrit ci-après.

En fonctionnement, le comparateur 21 compare la tension U_{CS} délivrée par le capteur photosensible 10 à une tension de seuil U_{ref}. Si la tension U_{CS} est strictement supérieure à la tension U_{ref}, le comparateur 21 commande la fermeture de l'interrupteur 22. Ainsi, la tension Uₑ aux bornes 31 du ou des obturateurs 30 est égale à la tension Ucs.

Dans le cas ou la tension Ucs est inférieure ou égale à la tension U_{ref}, le comparateur 21 commande l'ouverture de l'interrupteur 22, et l'obturateur 30 n'est plus alimenté.

En référence à la figure 1a, un mode de réalisation particulier du montage électronique du filtre optique 1 est représenté.

Dans ce mode de réalisation, la tension Uₑ délivrée à l'obturateur à cristaux liquides 30 est égale à la tension U_{CS} lorsque l'interrupteur 22 est fermé. Or, dans le cas d'un obturateur à cristaux liquides, par exemple de type nématique, il existe une tension de polarisation U_{LCD} minimum à appliquer à l'obturateur pour que l'orientation des molécules change et que l'obscurcissement soit effectif sur le verre.

En outre, pour que l'orientation des molécules des cristaux liquides change entièrement et de façon homogène, il faut que la tension appliquée soit supérieure à cette tension de polarisation, additionnée de quelques dixièmes de volts. En effet, si ce n'est pas le cas, les molécules des cristaux liquides ne s'orientent pas toutes de façon homogène, ce qui est à l'origine des irisations ou diaprures qui peuvent apparaître sur le verre et gêner l'usager.

Par conséquent, il faut que la tension U_{CS}, qui est transmise aux obturateurs 30 quand l'interrupteur 22 est fermé soit strictement supérieure à la tension de polarisation U_{LCD}, c'est-à-dire égale à la tension U_{LCD} de polarisation, additionnée de quelques dixièmes de volts, que l'on notera U_{LCD} + ε. Pour ce faire, la tension de référence U_{ref} du comparateur est choisie égale à U_{LCD} + ε. Par exemple, pour une tension U_{LCD} de 3V, la tension U_{ref} est choisie égale à 3,3V.

Selon un autre mode de réalisation de l'invention, représenté en figure 1b, le module électronique de commande comprend en outre un régulateur de tension 23, positionné entre l'interrupteur 22 et le ou les obturateurs à cristaux optiques 30.

Ce régulateur de tension 23 permet, dans le cas où l'opacité de l'obturateur 30 croit avec la tension appliquée, et particulièrement quand celle-ci est supérieure à la tension de polarisation U_{LCD}, de limiter la valeur de la tension Uₑ aux bornes des électrodes 310, 311 de l'obturateur 30, et ce afin de limiter l'opacification de l'obturateur. En effet, si le filtre optique selon l'invention est utilisé par exemple sous forme intégrée à des verres de lunettes de soleil, il est préférable de ne pas excéder un seuil d'opacité. Sinon, l'utilisateur des lunettes s'en trouverait gêné, voire il pourrait être mis en danger.

Selon un mode alternatif de réalisation de l'invention telle qu'illustrée en figure 1b, le régulateur de tension 23 peut être un régulateur élévateur de tension. Ainsi, la tension U_{ref} peut être choisie indépendamment de la valeur de la tension U_{LCD}. En particulier, elle peut être choisie inférieure à la tension U_{LCD}. Dans ce cas, l'interrupteur est fermé pour toute tension U_{CS} supérieure à la tension U_{ref}, ce qui peut comprendre des tensions inférieures à U_{LCD} + ε. Le régulateur de tension 23 reçoit donc cette tension Ucs et délivre en sortie une tension Uₑ, de préférence strictement supérieure à U_{LCD}, et même supérieure ou égale à U_{LCD} + ε, afin que les molécules des cristaux liquides puissent adopter une orientation homogène.

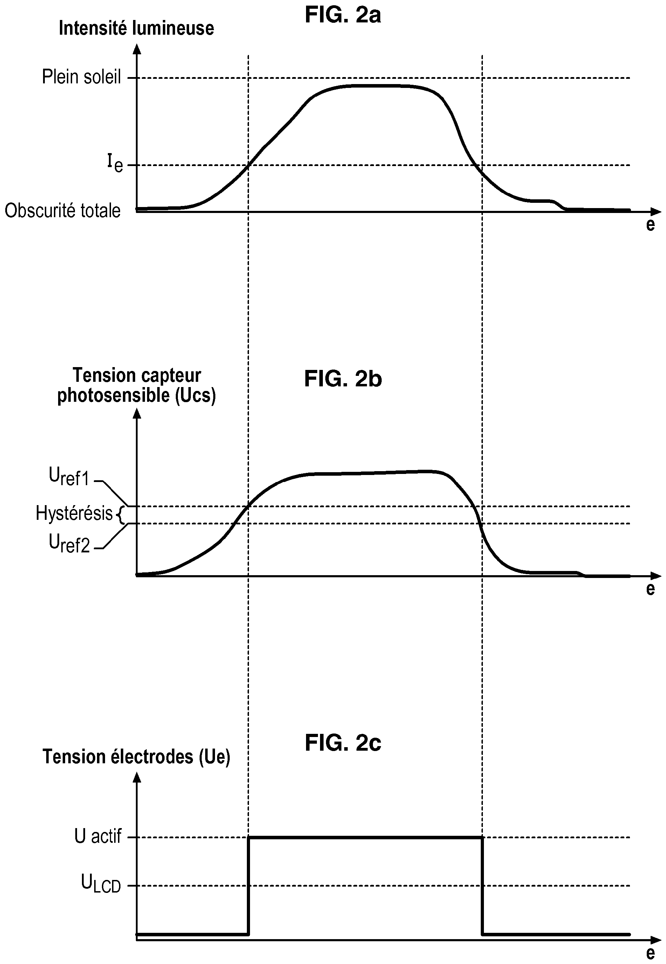

En référence à la figure 2, sont représentés des diagrammes de fonctionnement du filtre optique selon l'invention.

Sur le premier diagramme 2a est illustrée une variation de l'intensité lumineuse sur une fenêtre de temps donné. Cette intensité lumineuse varie depuis un état d'obscurité totale à un état très lumineux, analogue à la luminosité ambiante au soleil, en franchissant un niveau Iₑ, à partir duquel on peut être ébloui.

Sur le second diagramme 2b est représentée la tension Ucs délivrée par le capteur photosensible 10 au cours de l'évolution de la luminosité. La tension U_{CS} délivrée en fonction de la luminosité est ajustée par le constructeur, et elle peut être choisie de manière à ce que la tension U_{CS} délivrée au niveau du seuil d'éblouissement Iₑ soit strictement supérieure à la tension de seuil U_{ref}.

On constate que la tension U_{CS} évolue en même temps que l'intensité lumineuse.

Enfin sur le troisième diagramme 2c est illustrée la tension aux électrodes 310, 311 de l'obturateur à cristaux liquides 30. Au moment où la luminosité atteint le niveau d'éblouissement Iₑ, la tension U_{CS} atteint le seuil supérieur à U_{ref}, comme décrit ci-avant, et la transition au niveau des électrodes 310, 311 évite les risques d'irisation.

De retour à la figure 2b, on constate qu'en réalité le module électronique de commande 20 présente une hystérésis, entraînant que la tension de seuil par excès est différente de la tension de seuil par défaut. Nous appellerons dans la suite ces deux tensions de seuil U_{ref1} et U_{ref2}. Cette hystérésis permet de stabiliser le circuit. Des composants électroniques assurant une fonction de comparateur vis-à-vis de deux seuils différents respectivement par excès et par défaut existent et sont connus de l'homme de l'art. Ils ne seront donc pas décrits plus en détails par la suite..

En particulier, selon le mode de réalisation préféré pour lequel la tension U_{CS} délivrée par le capteur photosensible 10 croit avec l'intensité lumineuse, la tension de seuil d'activation, quand l'intensité lumineuse dépasse l'intensité d'éblouissement Iₑ, est la valeur maximale parmi les tensions de seuil U_{ref1} et U_{ref2}, et la tension de seuil de désactivation, quand l'intensité lumineuse devient inférieure au seuil d'éblouissement Iₑ, est la valeur minimale parmi les tensions U_{ref1} et U_{ref2}.

Par exemple, U_{ref1} est strictement supérieure à U_{ref2}, et U_{ref1} est alors la tension de seuil d'activation, et U_{ref2} est la tension de seuil de désactivation.

U_{ref1} et U_{ref2} sont alors distinctes de quelques dixièmes de volts, et en particulier elles sont choisies de préférence afin que la tension de polarisation U_{LCD} soit strictement inférieure à ces deux tensions. Par strictement comprise, on entend que U_{ref2} est supérieure à U_{LCD} additionnée de un ou quelques dixièmes de volts, et U_{ref1} est supérieure à U_{ref2} additionnée de un ou quelques dixièmes de volts.

Enfin, le niveau d'opacité OP₂ des verres, quand la tension Uₑ aux bornes de l'obturateur 30 est supérieure à la tension de polarisation U_{LCD}, peut être constant, et est alors défini à l'origine par le matériel utilisé, ou a posteriori par la définition de la tension de seuil U_{ref}.

Alternativement, le filtre optique peut en outre comprendre un dispositif de commande manuelle de l'opacité des verres, pour ajuster l'opacité OP₂ en fonction des souhaits des usagers. Par exemple, on peut utiliser un régulateur élévateur de tension, dont la tension de sortie est ajustable, afin d'ajuster la tension de seuil délivrée aux obturateurs à cristaux liquides.

Le filtre optique selon l'invention peut également comprendre un dispositif additionnel permettant de désactiver manuellement et le cas échéant de réactiver manuellement le module électronique de commande.

Cette invention comporte de nombreux avantages.

Tout d'abord, l'utilisation de seuils en tension permet d'éviter tout risque de diaprures liées à une mauvaise orientation des molécules dans les cristaux liquides, d'autant plus que ces diaprures sont généralement persistantes, même quand la tension aux bornes de l'obturateur à cristaux liquides redescend en dessous de la tension de polarisation.

En outre, puisque la seule source d'alimentation du module électronique de commande et de l'obturateur à cristaux liquides est le capteur photosensible, le filtre ne comporte pas de batterie qui pourrait faire défaut à l'usager pendant qu'il en a besoin. Le fonctionnement du filtre est uniquement conditionné à la présence ou non de soleil ou d'une autre source lumineuse.

Ce fonctionnement à seuil basé uniquement sur la tension de sortie du capteur photosensible permet encore une adaptation quasi-instantanée de l'obscurcissement du verre. Par quasi-instantané, on entend un temps de l'ordre du centième de seconde, et qui est en particulier inférieur au temps de persistance rétinienne. Ceci permet à l'usager de percevoir l'adaptation comme instantanée, et ce même pour des séquences de changement rapide de la luminosité, comme par exemple dans certaines tunnels.

Enfin, ce circuit électronique extrêmement simple du filtre optique est miniaturisable, et peut être inséré de façon très discrète dans une cavité d'un support 50.

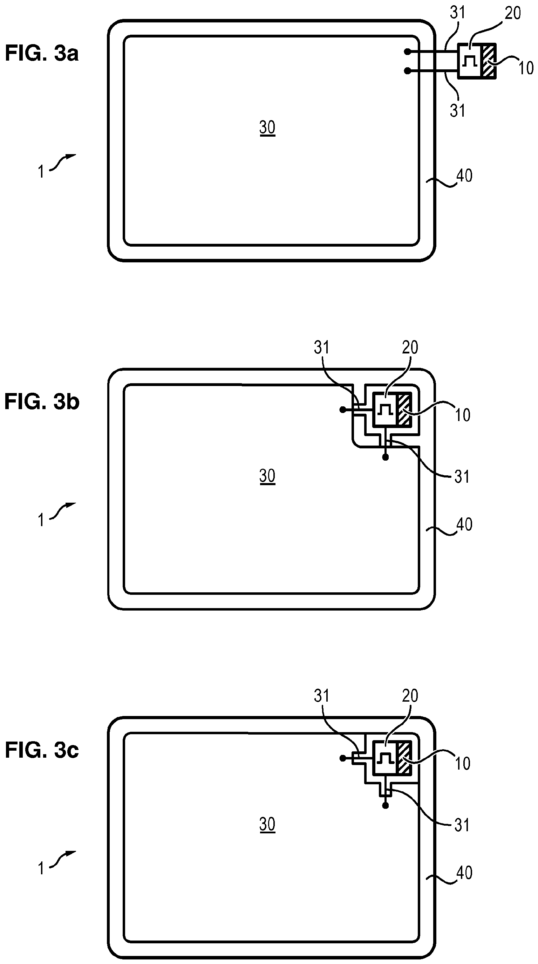

Quelque soit le support 50, deux configurations sont possibles pour l'intégration du filtre optique 1 au support. Selon un premier mode de réalisation, et en référence à la figure 3a, le capteur photosensible 10 et le module électronique de commande 20 peuvent être insérés dans le support 50 (non représenté dans la figure), et être connectés à l'obturateur 30, qui peut être intégré à un verre 40 par les électrodes 310, 311 et qui est connecté au module électronique 20 par ses bornes 31.

Alternativement, il est aujourd'hui possible d'utiliser une surface d'appui en verre ou en un autre matériau optiquement transparent (par exemple certains plastiques) pour intégrer l'ensemble du filtre optique 1, comme illustré en figure 3b et 3c.

Sur la figure 3b, le capteur photosensible 10 et le module électronique de commande 20 sont intégrés à une surface d'appui 40, sur lequel est disposé l'obturateur 30, sans recouvrir le capteur photosensible 10 ni le module électronique de commande 20.

Sur la figure 3c, le capteur photosensible 10 et le module électronique de commande 20 sont disposés sur une surface d'appui 40 ou intégrés à celle-ci, et sont recouverts par l'obturateur à cristaux liquides 30. Le cas échéant, les écrans optiquement transparents 330 et 331 de l'obturateur optique peuvent former le verre 40 servant de surface d'appui au filtre optique. Alternativement, une surface d'appui 40 peut être recouverte de l'obturateur à cristaux liquides 30.

Dans ces deux derniers cas, on utilise de préférence des capteurs photosensibles de type cellules photovoltaïques intégrées au verre. En outre, dans ce cas l'électronique de commande est de préférence réalisée dans un circuit imprimé réalisé par sérigraphie sur le verre.

En outre dans ces cas, l'obturateur à cristaux liquides 30 peut ne couvrir qu'une ou certaines parties de la surface d'appui 40.

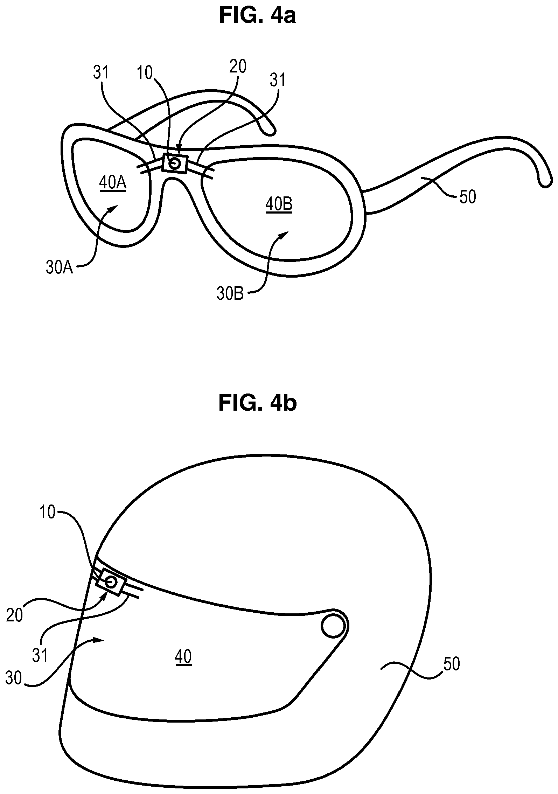

En référence à la figure 4a, le support 50 peut être une monture de lunettes comprenant deux verres 40A et 40B formant les surfaces d'appui 40 mentionnées ci-avant. On peut utiliser un capteur photosensible 10 suffisamment petit pour être intégré de façon très discrète à la monture 50 des lunettes, comme représenté sur la figure. En outre, le module électronique de commande est assez simple pour être miniaturisé et être lui aussi intégré dans la monture 50.

En outre, le filtre optique 1 comprend deux obturateurs à cristaux liquides 30A, 30B. Les écrans optiquement transparents 330A, 331A, 330B, 331B de l'obturateur 30 peuvent constituer les verres 40A, 40B des lunettes. Alternativement, on peut adjoindre aux obturateurs 30A, 30B au moins un verre additionnel 41, de sorte que les obturateurs 30A, 30B soient intégrés aux surfaces d'appui 40 des lunettes.

En outre, l'intégration d'un obturateur à cristaux liquides 30 dans des verres de lunettes 40A, 40B est combinable avec le fait que les verres apportent une correction optique au porteur.

Le cas échéant, et en référence à la figure 5, le verre additionnel 41 peut être une lame de verre travaillée selon des techniques connues pour apporter une correction optique au porteur, et placée en amont, ou éventuellement en aval de l'obturateur par rapport au trajet de la lumière incidente.

Alternativement, l'obturateur est placé entre deux lames de verres 41 taillées de façon à apporter une correction optique au porteur, les obturateurs étant alors courbés de façon à épouser les surfaces de contact des lames de verres.

En outre, et de retour à la figure 5, les verres 40 des lunettes peuvent de manière optionnelle comporter un ou plusieurs filtre anti-UV 42.

L'invention est applicable de la même manière au cas du monocle, qui ne comprend qu'un seul verre 40 et dans ce cas le filtre optique 1 ne comprend qu'un seul obturateur à cristaux liquides 30 intégré au verre.

Ainsi on obtient dans un produit unique une protection solaire qui se déclenche automatiquement en fonction du besoin, et une correction optique, qui elle est permanente. Ce produit unique élimine donc tout risque lié aux phases de transition rapides d'intensité lumineuse évoquées ci-avant.

En référence à la figure 4b, le support 51 peut être un casque, tel que par exemple un casque de véhicules à deux roues, le casque comprenant une visière formant le verre 40 du filtre optique 1.

En outre, les écrans optiquement transparents 330, 331 ne sont pas limités à des écrans en verre mais peuvent également être des écrans souples, par exemple en plastique souple, de manière à pouvoir être déformés pour présenter une courbure voulue.

Les applications du filtre optique selon l'invention ne sont toutefois pas limitées à ce mode de réalisation, mais peuvent également concerner des vitres utilisées dans des bâtiments, par exemple sur des fenêtres, des portes, ou des baies vitrées, ou tout type de surface vitrée dont l'obturation partielle, temporaire ou permanente, peut être réalisée à l'aide du filtre optique selon l'invention.

Quelque soit l'application du filtre optique, le caractère automatique de son activation le rend plus pratique et d'utilisation moins risquée qu'un dispositif de protection solaire traditionnel.

## Revendications

1. Filtre optique (1) comprenant :
- au moins un obturateur à cristaux liquides (30) optiquement transparent, présentant une tension de polarisation U_{LCD} formant un seuil entre deux états de polarisation, et adapté pour commuter entre au moins deux opacités OP₁ et OP₂, l'opacité OP₂ étant strictement supérieure à l'opacité OP₁, quand la tension qu'il reçoit est respectivement inférieure ou supérieure à la tension de polarisation U_{LCD} et
- un système d'électronique comprenant
∘ un module électronique de commande (20) de l'obturateur à cristaux liquides (30), adapté pour commander en tension l'obturateur à cristaux liquides (30),
∘ un capteur photosensible (10), adapté pour délivrer au module électronique de commande (20) une tension continue U_{CS} variable en fonction de l'intensité lumineuse qu'il reçoit, le capteur photosensible (10) étant la seule source d'alimentation du module électronique de commande (20) et de l'obturateur à cristaux liquides (30),
le filtre optique (1) étant **caractérisé en ce que**
- le module électronique de commande (20) comprend un comparateur de tension (21) et un interrupteur (22) lequel interrupteur est disposé entre le capteur photosensible et l'obturateur,
- et le module électronique de commande est adapté de sorte que si le capteur photosensible (10) reçoit une intensité lumineuse inférieure à un seuil d'éblouissement Iₑ, l'obturateur à cristaux liquides (30) présente une opacité OP₁, et
- le module électronique de commande (20) est adapté pour, lorsque le capteur photosensible (10) reçoit une intensité lumineuse supérieure au seuil d'éblouissement Iₑ, délivrer à l'obturateur à cristaux liquides (30) une tension continue Uₑ strictement supérieure à la tension de polarisation U_{LCD} de l'obturateur (30), de sorte que celui-ci commute de l'opacité OP₁ à l'opacité OP₂, dans lequel :
- le comparateur de tension (21) compare la tension U_{CS} délivrée par le capteur photosensible (10) à une tension de seuil par excès U_{ref1} ou à une tension de seuil par défaut U_{ref2}, les tensions U_{ref1} et U_{ref2} étant telles que U_{ref1} est strictement supérieure à U_{ref2}, et U_{ref2} est strictement supérieure à la tension de polarisation U_{LCD} de l'obturateur,
- lorsque le filtre optique comprend plus d'un obturateur à cristaux liquides, tous les obturateurs à cristaux liquides sont connectés en parallèle à la sortie de l'interrupteur afin de recevoir tous la même tension d'entrée,
- si la tension U_{CS} délivrée par le capteur (10) dépasse la tension de seuil par excès U_{ref1}, lorsque le capteur photosensible (10) reçoit une intensité lumineuse supérieure au seuil d'éblouissement Iₑ, le module électronique est adapté pour fermer l'interrupteur (22) de sorte que l'interrupteur délivre à l'obturateur à cristaux liquides (30) une tension continue Uₑ égale à la tension U_{CS} et supérieure à la tension de polarisation U_{LCD} de l'obturateur (30) additionnée de quelques dixièmes de volts, et l'opacité de l'obturateur à cristaux liquides (30) devient égale à OP2,
- si la tension délivrée par le capteur photosensible (10) U_{CS} dévient inférieure à la tension de seuil par défaut U_{ref2} lorsque le capteur photosensible (10) reçoit une intensité lumineuse inférieure au seuil d'éblouissement Iₑ, l'interrupteur (22) s'ouvre, l'obturateur à cristaux liquides (30) n'est plus alimenté et son opacité devient égale à l'opacité OP₁.

2. Filtre optique selon l'une quelconque des revendications précédentes, dans lequel le filtre optique comprend en outre un régulateur de tension (23) adapté pour limiter la tension appliquée à l'obturateur, positionné en aval de l'interrupteur (22), et adapté pour délivrer une tension Uₑ maximale constante et strictement supérieure à la tension de polarisation U_{LCD} de l'obturateur (30).

3. Filtre optique selon l'une des revendications 1 ou 2, dans lequel l'opacité OP₂ est constante pour toute tension Uₑ strictement supérieure à la tension de polarisation U_{LCD}.

4. Filtre optique selon la revendication précédente, comprenant en outre un dispositif de réglage manuel de l'opacité OP₂ de l'obturateur à cristaux liquides (30).

5. Filtre optique selon l'une des revendications précédentes, comprenant en outre un dispositif de désactivation manuelle permanente du module électronique de commande (20).

6. Filtre optique selon l'une des revendications précédentes, dans lequel le capteur photosensible (10) est intégré à au moins une surface d'appui (330, 331, 40), et le module électronique de commande est réalisé sur un circuit imprimé par sérigraphie sur au moins un verre (330, 331, 40).

7. Filtre optique selon l'une des revendications précédentes, dans lequel l'obturateur à cristaux liquides (30) est intégré dans une zone partielle d'un verre (40).

8. Paire de lunettes comprenant un filtre optique selon l'une des revendications précédentes, le filtre optique comprenant deux obturateurs à cristaux liquides (30A, 30B) adaptés pour former des verres ou pour être intégrés aux verres (40A, 40B) des lunettes.

9. Paire de lunettes selon la revendication précédente, comprenant en outre au moins un verre (41) taillé ou traité de manière à apporter une correction optique à un sujet porteur.

10. Casque comprenant un filtre optique selon l'une des revendications 1 à 9, l'obturateur à cristaux liquides (30) du filtre optique (1) étant adapté pour former ou être intégré à une visière (40) du casque.

## Patentansprüche

1. Optischer Filter (1), umfassend:
- mindestens eine optisch transparente Flüssigkeitskristallblende (30), aufweisend eine Polarisationsspannung U_{LCD}, die einen Grenzwert zwischen zwei Polarisationszuständen bildet und ausgebildet ist, um zwischen mindestens zwei Opazitäten OP₁ und OP₂ umzuschalten, wobei die Opazität OP₂ strikt größer als die Opazität OP₁ ist, wenn die Spannung, die sie erhält, jeweils kleiner oder größer als die Polarisationsspannung U_{LCD} ist, und
- ein elektronisches System, umfassend
∘ ein elektronisches Steuermodul (20) der Flüssigkeitskristallblende (30), das ausgebildet ist, um die Spannung der Flüssigkeitskristallblende (30) zu steuern,
∘ einen photosensiblen Sensor (10), der ausgebildet ist, um dem elektronischen Steuermodul (20) eine Gleichspannung U_{CS} bereitzustellen, die in Abhängigkeit von der Lichtstärke, die er erhält, variabel ist, wobei der photosensible Sensor (10) die einzige Versorgungsquelle des elektronischen Steuermoduls (20) und der Flüssigkeitskristallblende (30) ist,
wobei der optische Filter (1) **dadurch gekennzeichnet ist, dass**
- das elektronische Steuermodul (20) einen Spannungskomparator (21) und einen Schalter (22) umfasst, wobei der Schalter zwischen dem photosensiblen Sensor und der Blende angeordnet ist,
- und das elektronische Steuermodul derart ausgebildet ist, dass, wenn der photosensible Sensor (10) eine Lichtstärke unter einem Blendungsgrenzwert Iₑ erhält, die Flüssigkeitskristallblende (30) eine Opazität OP₁ aufweist, und
- das elektronische Steuermodul (20) ausgebildet ist, dass, wenn der photosensible Sensor (10) eine Lichtstärke über dem Blendungsgrenzwert Iₑ erhält, der Flüssigkeitskristallblende (30) eine Gleichspannung Uₑ strikt größer als die Polarisationsspannung U_{LCD} der Blende (30) bereitgestellt wird, so dass diese von der Opazität OP₁ in die Opazität OP₂ umschaltet, wobei:
- der Spannungskomparator (21) die von dem photosensiblen Sensor (10) bereitgestellte Spannung U_{CS} mit einer Überschuss-Grenzwertspannung U_{ref1} oder mit einer Standard-Grenzwertspannung U_{ref2} vergleicht, wobei die Spannungen U_{ref1} und U_{ref2} derart sind, dass U_{ref1} strikt größer als U_{ref2} ist und U_{ref2} strikt größer als die Polarisationsspannung U_{LCD} der Blende ist,
- wenn der optische Filter mehr als eine Flüssigkeitskristallblende umfasst, alle Flüssigkeitskristallblenden parallel mit dem Ausgang des Schalters verbunden sind, damit alle dieselbe Eingangspannung erhalten,
- wenn die vom Sensor (10) bereitgestellte Spannung U_{CS} über der Überschuss-Grenzwertspannung U_{ref1} liegt, wenn der photosensible Sensor (10) eine Lichtstärke über dem Blendungsgrenzwert Iₑ erhält, das elektronische Modul ausgebildet ist, um den Schalter (22) zu schließen, so dass der Schalter der Flüssigkeitskristallblende (30) eine Gleichspannung Uₑ bereitstellt, die gleich der Spannung U_{CS} ist und größer als die Polarisationsspannung U_{LCD} der Blende (30), plus einige Zehntel Volt, und die Opazität der Flüssigkeitskristallblende (30) gleich OP₂ wird,
- wenn die vom photosensiblen Sensor (10) bereitgestellte Spannung U_{CS} unter die Standard-Grenzwertspannung U_{ref2} abfällt, wenn der photosensible Sensor (10) eine Lichtstärke unter dem Blendungsgrenzwert Iₑ erhält, sich der Schalter (22) öffnet, die Flüssigkeitskristallblende (30) nicht mehr versorgt wird und ihre Opazität gleich der Opazität OP₁ wird.

2. Optischer Filter nach einem der vorangehenden Ansprüche, wobei der optische Filter ferner einen Spannungsregler (23) umfasst, der ausgebildet ist, um die an der Blende anliegende Spannung zu begrenzen, der nach dem Schalter (22) positioniert ist, und ausgebildet um eine konstante maximale Spannung Uₑ bereitzustellen, die strikt größer als die Polarisationsspannung U_{LCD} der Blende (30) ist.

3. Optischer Filter nach einem der Ansprüche 1 oder 2, wobei die Opazität OP₂ für jede Spannung Uₑ strikt größer als die Polarisationsspannung U_{LCD} konstant ist.

4. Optischer Filter nach vorangehendem Anspruch, umfassend ferner eine Vorrichtung zur manuellen Einstellung der Opazität OP₂ der Flüssigkeitskristallblende (30).

5. Optischer Filter nach einem der vorangehenden Ansprüche, umfassend ferner eine Vorrichtung zur ständigen manuellen Deaktivierung des elektronischen Steuermoduls (20).

6. Optischer Filter nach einem der vorangehenden Ansprüche, wobei der photosensible Sensor (10) in mindestens eine Stützfläche (330, 331, 40) integriert ist, und das elektronische Steuermodul auf einer auf mindestens einem Glas (330, 331, 40) gedruckten Siebdruckschaltung ausgeführt ist.

7. Optischer Filter nach einem der vorangehenden Ansprüche, wobei die Flüssigkeitskristallblende (30) in einen Teilbereich eines Glases (40) integriert ist.

8. Brille, umfassend einen optischen Filter nach einem der vorangehenden Ansprüche, wobei der optische Filter zwei Flüssigkeitskristallblenden (30A, 30B) umfasst, die ausgebildet sind, um Gläser zu bilden oder um in Brillengläser (40A, 40B) integriert zu sein.

9. Brille nach vorangehendem Anspruch, umfassend ferner mindestens ein Glas (41), das geschliffen ist oder derart bearbeitet, dass ein Träger eine optische Korrektur erhält.

10. Helm, umfassend einen optischen Filter nach einem der Ansprüche 1 bis 9, wobei die Flüssigkeitskristallblende (30) des optischen Filters (1) ausgebildet ist, um einen Schirm (40) des Helms zu bilden oder darin integriert zu sein.

## Claims

1. An optical filter (1) comprising:
- at least one optically transparent liquid-crystal shutter (30), having a polarisation voltage U_{LCD} forming a threshold between two states of polarisation, and adapted to switch between at least two opacities OP₁ and OP₂, the opacity OP₂ being strictly greater than the opacity OP₁, when the voltage which it receives is respectively less or greater than the polarisation voltage U_{LCD} and
- an electronics system comprising
∘ an electronic control module (20) of the liquid-crystal shutter (30), adapted to control the voltage of the liquid-crystal shutter (30),
∘ a photosensitive sensor (10), adapted to provide the electronic control module (20) with a continuous voltage U_{CS} variable as a function of the luminous intensity which it receives, the photosensitive sensor (10) being the sole source of supply of the electronic control module (20) and of the liquid-crystal shutter (30),
the optical filter (1) being **characterised in that**
- the electronic control module (20) comprises a voltage comparator (21) and an interrupter (22), said interrupter is disposed between the photosensitive sensor and the shutter,
- and the electronic control module is adapted such that if the photosensitive sensor (10) receives a luminous intensity less than a dazzle threshold Iₑ, the liquid-crystal shutter (30) has an opacity OP₁, and
- the electronic control module (20) is adapted so that, when the photosensitive sensor (10) receives a luminous intensity greater than the dazzle threshold Iₑ, to provide the liquid-crystal shutter (30) with a continuous voltage Uₑ strictly greater than the polarisation voltage U_{LCD} of the shutter (30), such that the latter switches from the opacity OP₁ to the opacity OP₂, wherein:
- the voltage comparator (21) compares the voltage U_{CS} delivered by the photosensitive sensor (10) to a threshold voltage by excess U_{ref1} or to a threshold voltage by default U_{ref2}, the voltages U_{ref1} and U_{ref2} being such that U_{ref1} is strictly greater than U_{ref2}, and U_{ref2} is strictly greater than the polarisation voltage U_{LCD} of the shutter,
- when the optical filter comprises more than a liquid-crystal shutter, all the liquid-crystal shutters are connected in parallel to the output of the interrupter to all receive the same input voltage,
- if the voltage U_{CS} delivered by the sensor (10) exceeds the threshold voltage by excess U_{ref1} when the photosensitive sensor (10) receives a luminous intensity greater than the dazzle threshold Iₑ, the electronic module is adapted to close the interrupter (22) so that the interrupter provides the liquid-crystal shutter (30) with a continuous voltage Uₑ equal to the voltage U_{CS} and greater than the polarisation voltage U_{LCD} of the shutter (30) with a few tenths of volts added, and the opacity of the liquid-crystal shutter (30) becomes equal to OP₂,
- if the voltage delivered by the photosensitive sensor (10) U_{CS} becomes less than the threshold voltage by default U_{ref2} when the photosensitive sensor (10) receives a luminous intensity less than the dazzle threshold Iₑ, the interrupter (22) opens, the liquid-crystal shutter (30) is no longer fed and its opacity becomes equal to the opacity OP₁.

2. The optical filter according to any one of the preceding claims, wherein the optical filter further comprising a voltage regulator (23), adapted to limit the voltage applied to the shutter, positioned downstream of the interrupter (22), and adapted to deliver a voltage Uₑ which is maximum constant and strictly greater than the polarisation voltage U_{LCD} of the shutter (30).

3. The optical filter according to one of claims 1 or 2, wherein the opacity OP₂ is constant for any voltage Uₑ strictly greater than the polarisation voltage U_{LCD}.

4. The optical filter according to the preceding claim, further comprising a device for manual adjustment of the opacity OP₂ of the liquid-crystal shutter (30).

5. The optical filter according to one of the preceding claims, further comprising a device for permanent manual deactivation of the electronic control module (20).

6. The optical filter according to one of the preceding claims, wherein the photosensitive sensor (10) is integrated with at least one bearing surface (330, 331, 40), and the electronic control module is made on a printed circuit by silkscreen printing on at least one lens (330, 331, 40).

7. The optical filter according to one of the preceding claims, wherein the liquid-crystal shutter (30) is integrated into a partial zone of a lens (40).

8. A pair of glasses comprising an optical filter according to one of the preceding claims, the optical filter comprising two liquid-crystal shutters (30A, 30B) adapted to form glasses or be integrated into the lenses (40A, 40B) of the glasses.

9. The pair of glasses according to the preceding claim, further comprising at least one glass (41) machined or processed so as to lend optical correction to a wearer.

10. A helmet comprising an optical filter according to one of claims 1 to 9, the liquid-crystal shutter (30) of the optical filter (1) being adapted to form or be integrated into a visor (40) of the helmet.
